# EUROPEAN PATENT SPECIFICATION

(11) **EP 3 768 096 B1**
(45) Date of publication and mention of the grant of the patent: **11.11.2026**
(21) Application number: 19717644.9
(22) Date of filing: 22.03.2019
(51) Int. Cl.: A23K 20/142, A23K 50/40, A23K 50/42, A23K 50/48, A61K 31/195, A61K 31/198

(54) **REDUCED METHIONINE DIET FOR DOGS**
VERKÜRZTE METHIONINDIÄT FÜR HUNDE
RÉGIME ALIMENTAIRE À MÉTHIONINE RÉDUITE POUR CHIENS

(30) Priority: 23.03.2018 GB 201804698
(43) Date of publication of application: 27.01.2021
(73) Proprietor: MARS, INCORPORATED, McLean, Virginia 22101 (US)
(72) Inventor: ALLAWAY, David, Leicestershire LE14 4RT (GB); HARRISON, Matthew James, Leicestershire LE14 4RT (GB); GRAY, Kerry, Leicestershire LE14 4RT (GB); COLYER, Alison, Leicestershire LE14 4RT (GB)
(74) Representative: Greaves Brewster LLP
(86) International application number: PCT/US2019/023700
(87) International publication number: WO 2019/183557

(56) References cited:
- WO-A1-00/44375
- US-A1- 2005 014 698
- CHRISTOPH RUCKENSTUHL ET AL: "Lifespan Extension by Methionine Restriction Requires Autophagy-Dependent Vacuolar Acidification", PLOS GENETICS, vol. 10, no. 5, 1 May 2014 (2014-05-01), pages e1004347, XP055589250, DOI: 10.1371/journal.pgen.1004347
- ELSHORBAGY AMANY K ET AL: "Effect of taurine and N-acetylcysteine on methionine restriction-mediated adiposity resistance", METABOLISM, vol. 62, no. 4, 25 September 2010 (2010-09-25), pages 509 - 517, XP028596650, ISSN: 0026-0495, DOI: 10.1016/J.METABOL.2012.10.005
- L. SUN ET AL: "Life-Span Extension in Mice by Preweaning Food Restriction and by Methionine Restriction in Middle Age", JOURNALS OF GERONTOLOGY, SERIES A, BIOLOGICAL SCIENCES ANDMEDICAL SCIENCES, vol. 64A, no. 7, 4 May 2009 (2009-05-04), US, pages 711 - 722, XP055589247, ISSN: 1079-5006, DOI: 10.1093/gerona/glp051
- RICHARD A. MILLER ET AL: "Methionine-deficient diet extends mouse lifespan, slows immune and lens aging, alters glucose, T4, IGF-I and insulin levels, and increases hepatocyte MIF levels and stress resistance : Methionine restriction slows mouse aging, R. A. Miller et al.", AGING CELL, vol. 4, no. 3, 13 April 2005 (2005-04-13), GB, pages 119 - 125, XP055589269, ISSN: 1474-9718, DOI: 10.1111/j.1474-9726.2005.00152.x
- GOMEZ A ET AL: "Cysteine dietary supplementation reverses the decrease in mitochondrial ROS production at complex I induced by methionine restriction", JOURNAL OF BIOENERGETICS AND BIOMEMBRANES, PLENUM PUBLISHING, NEW YORK, NY, US, vol. 47, no. 3, 15 March 2015 (2015-03-15), pages 199 - 208, XP035499897, ISSN: 0145-479X, [retrieved on 20150315], DOI: 10.1007/S10863-015-9608-X
- AMANY K. ELSHORBAGY ET AL: "Cysteine supplementation reverses methionine restriction effects on rat adiposity: significance of stearoyl-coenzyme A desaturase", JOURNAL OF LIPID RESEARCH, vol. 52, no. 1, 25 September 2010 (2010-09-25), US, pages 104 - 112, XP055589299, ISSN: 0022-2275, DOI: 10.1194/jlr.M010215
- BYUNG CHEON LEE ET AL: "Methionine restriction extends lifespan of Drosophila melanogaster under conditions of low amino-acid status", NATURE COMMUNICATIONS, vol. 5, no. 1, 7 April 2014 (2014-04-07), XP055589264, DOI: 10.1038/ncomms4592
- RAFAL KOZIEL ET AL: "Methionine restriction slows down senescence in human diploid fibroblasts", AGING CELL, vol. 13, no. 6, 1 October 2014 (2014-10-01), GB, pages 1038 - 1048, XP055589251, ISSN: 1474-9718, DOI: 10.1111/acel.12266
- CHRISTOPH RUCKENSTUHL ET AL: "Lifespan Extension by Methionine Restriction Requires Autophagy-Dependent Vacuolar Acidification", PLOS GENETICS, vol. 10, no. 5, 1 May 2014 (2014-05-01), pages e1004347, XP055589262, DOI: 10.1371/journal.pgen.1004347
- BYUNG CHEON LEE ET AL: "Methionine restriction and life-span control : Methionine restriction and life-span control", ANNALS OF THE NEW YORK ACADEMY OF SCIENCES, vol. 1363, no. 1, 10 December 2015 (2015-12-10), US, pages 116 - 124, XP055585826, ISSN: 0077-8923, DOI: 10.1111/nyas.12973
- PEREZ-GARCIA O ET AL: "Heterotrophic cultures of microalgae: Metabolism and potential products", WATER RESEARCH, ELSEVIER, AMSTERDAM, NL, vol. 45, no. 1, 1 January 2011 (2011-01-01), pages 11 - 36, XP027536678, ISSN: 0043-1354, [retrieved on 20100827]
- TORRES JOANA ET AL: "New therapeutic avenues in ulcerative colitis: thinking out of the box", GUT MICROBIOTA, vol. 62, no. 11, 8 October 2013 (2013-10-08), UK, pages 1642 - 1652, XP093132788, ISSN: 0017-5749, DOI: 10.1136/gutjnl-2012-303959
- PRÖSCHOLD THOMAS ET AL: "Chlamydomonas schloesseri sp. nov. (Chlamydophyceae, Chlorophyta) revealed by morphology, autolysin cross experiments, and multiple gene analyses", PHYTOTAXA, vol. 362, no. 1, 23 July 2018 (2018-07-23), pages 21, XP093132790, ISSN: 1179-3155, Retrieved from the Internet <URL:https://biotaxa.org/Phytotaxa/article/viewFile/36804/34167> DOI: 10.11646/phytotaxa.362.1.2
- SAKURAI KENTA ET AL: "Detection and characterization of phosphatidylcholine in various strains of the genusChlamydomonas(Volvocales, Chlorophyceae)", JOURNAL OF PLANT RESEARCH, TOKYO, JP, vol. 127, no. 5, 20 June 2014 (2014-06-20), pages 641 - 650, XP035379410, ISSN: 0918-9440, [retrieved on 20140620], DOI: 10.1007/S10265-014-0644-0
- R. SCOTT MCISAAC ET AL: "From yeast to human: exploring the comparative biology of methionine restriction in extending eukaryotic life span : Comparative biology of methionine restriction", ANNALS OF THE NEW YORK ACADEMY OF SCIENCES, vol. 1363, no. 1, 1 January 2016 (2016-01-01), US, pages 155 - 170, XP055585828, ISSN: 0077-8923, DOI: 10.1111/nyas.13032
- SHERRY SANDERSON: "Effects of dietary fat and L-carnitine on plasma and whole blood taurine concentrations and cardiac function in healthy dogs fed protein-restricted diets", AMERICAN JOURNAL OF VETERINARY RESEARCH, vol. 62, no. 10, 1 October 2001 (2001-10-01), pages 1616 - 1623, XP055585841
- BROWN-BORG HOLLY M ET AL: "Cutting back on the essentials: Can manipulating intake of specific amino acids modulate health and lifespan?", AGEING RESEARCH REVIEWS, ELSEVIER, AMSTERDAM, NL, vol. 39, 26 August 2016 (2016-08-26), pages 87 - 95, XP085165942, ISSN: 1568-1637, DOI: 10.1016/J.ARR.2016.08.007
- KWANG S KO ET AL: "The Journal of Nutrition Nutrient Physiology, Metabolism, and Nutrient-Nutrient Interactions Differences in Taurine Synthesis Rate among Dogs Relate to Differences in Their Maintenance Energy Requirement 1-3 Department of 4 Molecular", 1 January 2007 (2007-01-01), pages 1171 - 1175, XP055585836, Retrieved from the Internet <URL:https://academic.oup.com/jn/article-pdf/137/5/1171/23899181/1171.pdf> [retrieved on 20190515]

## Description

### Field

The present invention relates to diet compositions dogs, as well as to methods for preparing these compositions.

### Background

Of the 20 canonical amino acids, methionine and cysteine are the principal sulphur containing units. Other group members include homocysteine and taurine. However, only methionine and cysteine are incorporated into proteins.

Methionine is an 'essential amino acid' and therefore must be obtained directly from the diet. Methionine is a methyl donor, a constituent of proteins, and forms part of a coenzyme S-adenosylmethionine (SAM) which is responsible for over 40 known metabolic reactions involving transfer of methyl groups. Methionine is also a sulphur amino acid, and serves as a precursor for cysteine (non-essential amino acid), which is an important component of proteins such as glutathione and taurine.

Although methionine is essential, methionine-restricted diets (i.e. diets containing a low, but sufficient, level of methionine) have been reported to support health and longevity across a range of species (Lee et al., (2016) Ann N Y Acad Sci. 1363:116-24; McIsaac et al., (2016) Ann N Y Acad Sci. 1363:155-70). There is still debate about how or whether these perceived benefits are attributable to "protein-restriction" (Brown-Borg & Buffenstein, (2017) Ageing Res Rev. 39:87-95).

The challenge to application of methionine restriction in dogs is the concern over the health implications for dogs prone to taurine deficiency and subsequent potential dilated cardiomyopathy (DCM).

One specific issue in dogs related to diets low in sulfur-amino acids is a reduction in the amount of taurine synthesis (a product of S-amino acid metabolism). Under normal circumstances dogs are able to synthesise sufficient taurine from the sulphur-containing amino acids methionine and cysteine, therefore dogs fed diets containing adequate amounts of these constituents have normal taurine levels and demonstrate no signs of taurine deficiency, even with taurine absent in the diet. Low taurine availability can lead to increased risk of DCM, which in extreme cases can lead to death. Evidence indicates that large dogs are of particular concern, with marginal taurine compared to small dogs when fed a diet containing reduced sulphur amino acids (K.S. Ko et al., The Journal of Nutrition, 2007, 137 (5) p1171-1175).

In adult humans, equivalent amounts of methionine and cysteine are utilized for the re-synthesis of degraded proteins and peptides. Catabolism of methionine and cysteine is restricted at low intakes of sulphur amino acids, with incorporation of methionine and cysteine into protein having priority over glutathione and taurine production.

These pathways have not been directly studied in the dog, and it is not necessarily expected that the results are translatable between species. Old studies, dating back to the 1940s, have indicated that the total sulphur amino acid requirement in a dog diet was in the range of 2-4g/kg diet⁻¹. More recently, it has been reported that consumption of diets low in methionine and cysteine resulted in reduced taurine synthesis in large dogs with low energy requirements (Ko et al. supra.). Inadequate taurine levels are a known contributing factor to the development of dilated cardiomyopathy in dogs susceptible to the disease, although this relationship has not been proven to exist in other species.

Furthermore, taurine addition to pet diets is also known, in particular in combination with Vitamin C to enhance plasma Vitamin E levels (WO0044375). Addition of an adequate intake (generally about 1000mg taurine Kg diet (dry matter)⁻¹) of taurine to dog and cat diets low in protein or sulfur amino acids, in order to maintain body taurine pools has been reported (National Research Council, 2006. Nutrient requirements of dogs and cats. National Academies Press. Chapter 6, p135).

A study (Sanderson et al., (2001) American Journal of Veterinary Research 62 (10), p1616-1623) noted that feeding a diet containing 1.3 g/1000 kcal total sulphur amino acids in the context of a low-fat diet was sufficient to maintain beagles for 4 years without any apparent abnormalities. However, feeding 1.2g/1000 kcal combined with a higher-fat diet resulted in one dog exhibiting DCM as a consequence of taurine deficiency. This was almost completely corrected by 3 months of taurine supplementation, although the dog did go on to develop a murmur and was found to have mitral valve endocarditis.

As a result of this study, the National Research Council (NRC) suggested minimum for maintenance for total sulphur amino acids for dogs is 1.63g/1000kcal of total sulphur amino acids (methionine and cysteine) with at least 0.83g/1000kcal of methionine in a diet formulated to be fed at 130kcal/Kg body weight. In the light of this, many manufacturers of dog food ensure that levels of methionine well in excess of this amount are present to account for reductions in recommended energy requirements. US2005/014698 discloses food compositions with increased levels of sulfur-containing amino acids.

Free methionine may be added to dog products during diet formulation. However, this may have a significant cost implication. Affordable nutrition for companion animals such as dogs is important, in particular in developing markets, where pet nutrition may be of low priority to some households. As a result, development of reduced methionine diets which maintain or promote health are desirable.

### Summary

There has now been discovered a dietary range of methionine that is lower than current (recommended) dietary inclusion levels, that meets the requirements for the dog, is safe to consume, and that produced biological responses expected to lead to improved health and/or increased lifespan.

Accordingly, there is herein provided a diet composition for an adult dog. The invention is set out in the appended set of claims. The composition comprises a combination of methionine and cysteine.

The diet composition may further comprise an amount of taurine, e.g., the diet composition may comprise at least 0.125g/1000kcal, at least 0.25g/1000kcal taurine, but no more than 0.5g/1000kcal taurine. The diet composition may also comprise choline.

The diet composition may be in a dry or wet format. If provided as a dry food, the diet composition may comprise a kibble, which may optionally be coated. Dry foods may typically have moisture contents of less than 12% by weight of water, while wet foods typically have greater than 12% moisture by weight. In those instances wherein the diet composition is a wet food, the diet composition may be in in the form of a 'solids-in-gravy' type composition.

The combined ingredients may, in some instances, provide choline and/or an amount of taurine of at least 0.125g/1000kcal, at least 0.25g/1000kcal taurine, but no more than 0.5g/1000kcal taurine.

In some instances, the combined ingredients may provide a lesser amount of methionine, cysteine or taurine, with the remainder provided by adding methionine, taurine and/or cysteine or a cysteine providing derivative thereof during or after the combining process so as to produce a diet composition having the desired amounts. This may be the case, e.g., when the diet composition includes vegetable protein, as is contemplated for some embodiments.

It has now been discovered that a diet composition including restricted amounts of methionine may provide certain health and/or longevity benefits when fed to a companion animal, while also protecting dogs from dilated cardiomyopathy. And so, methods of promoting longevity in a dog, treating dilated cardiomyopathy or preventing cardiomyopathy in a dog that may be prone to develop the same are also described. The methods include feeding a dog the diet composition provided herein.

### Brief Description of the Drawings

The invention will now be particularly described by way of example with reference to the accompanying diagrammatic drawings in which:
Figure 1 is a graph showing the average daily methionine intake (g/KgMetBW) by week for dogs on the trial and coloured by diet. (In all figures, error bars represent 95% Confidence Intervals).
Figure 2 is a graph showing the bodyweights (kg) by week of dogs on the trial and coloured by diet.
Figure 3 is a graph showing cholesterol levels (mmol/L), with significant changes between diets within a week, by week for dogs on the trial and coloured by diet. Dotted lines indicate reference ranges, Idexx for biochemistry.

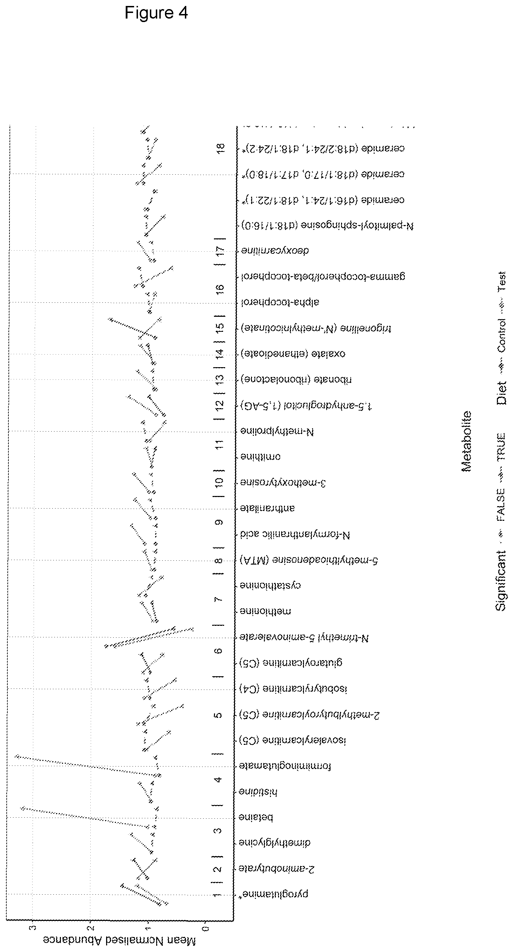

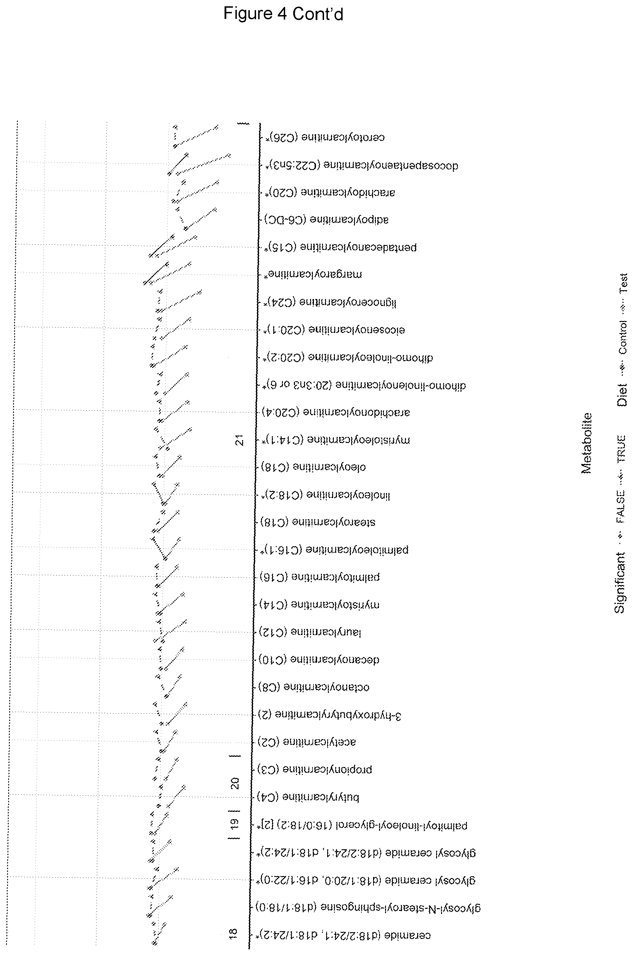

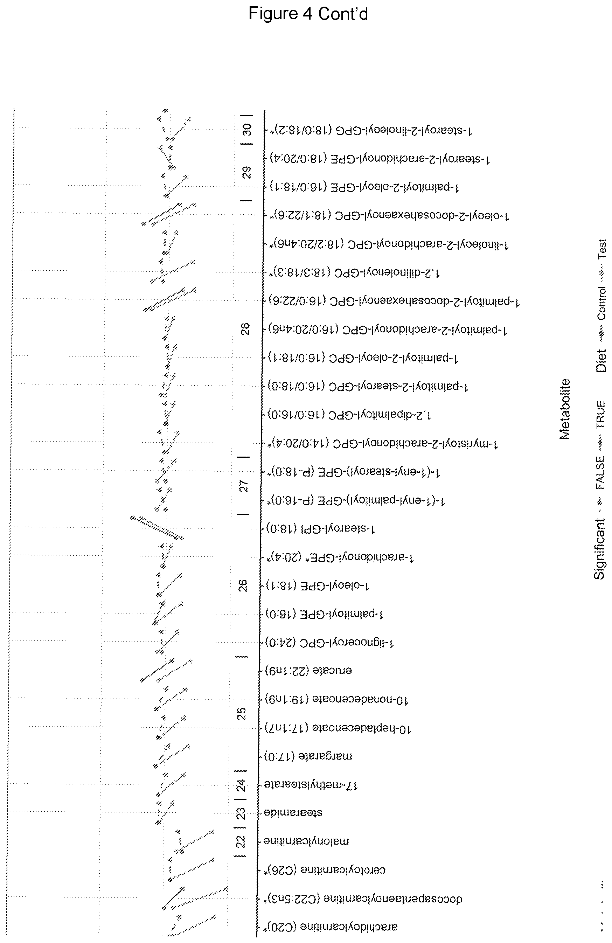

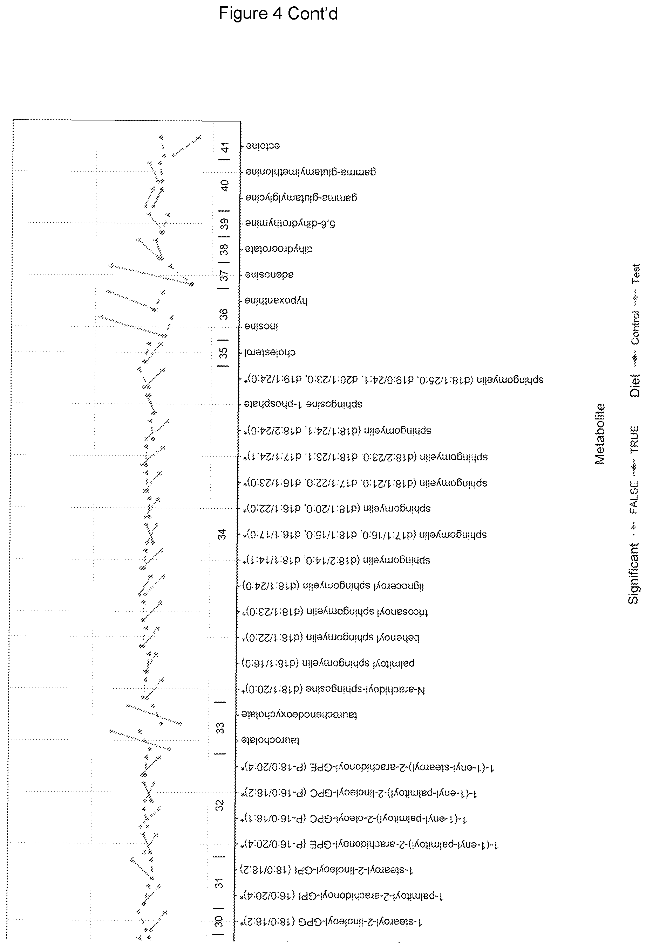

Figure 4 is a graph illustrating the levels of metabolites noted as showing significant differences of a metabolomic analysis of plasma of dogs on the trial, indicating fasted plasma metabolite fold-change relative to a normalised pool. Data are from baseline and after 32 weeks (left and right symbols respectively). Where the control group (diet A) are shown in black and the test group (diet B) in grey. Significant differences between diets at a time point are represented by triangle, significant differences between time points within diet are represented by solid lines connecting the two points.
Figure 5 is a graph showing the results of metabolomic analysis of samples from dogs for methionine levels against time in weeks, for control (dogs fed diet A throughout) (dotted lines) and test samples (dogs switched to diet B after the baseline sample) where error bars represent 95% confidence intervals.
Figure 6 is a graph showing the results of metabolomic analysis of samples from dogs for (A) Cysteine and (B) cystathionine levels against time in weeks, where error bars represent 95% confidence intervals.
Figure 7 is a graph showing the results of metabolomic analysis of samples from dogs for 5-methylthioadenosine levels against time in weeks, where error bars represent 95% confidence intervals.
Figure 8 is a graph showing the results of metabolomic analysis of samples from dogs for betaine levels against time in weeks, where error bars represent 95% confidence intervals.
Figure 9 is a graph showing the results of metabolomic analysis of samples from dogs for dimethylglycine levels against time in weeks where error bars represent 95% confidence intervals.
Figure 10 is a graph showing the results of metabolomic analysis of samples from dogs for sarcosine levels against time in weeks where error bars represent 95% confidence intervals.
Figure 11 is a graph showing the results of metabolomic analysis of samples from dogs for acetylcarnitine (C2) levels against time in weeks where error bars represent 95% confidence intervals.
Figure 12 is a graph showing the results of metabolomic analysis of samples from dogs for palmitoylcarnitine (C16) levels against time in weeks where error bars represent 95% confidence intervals.
Figure 13 is a graph showing the results of metabolomic analysis of samples from dogs for pentadecanoylcarnitine (C15) levels against time in weeks where error bars represent 95% confidence intervals.

### Detailed Description

As used herein, the expression 'adult dog' refers to a dog who is no longer growing, for example is at least 18 months or two years old, for example from 2 to 11 years old.

The composition of the invention provides a healthy diet composition for adult dogs, in spite of the fact that the amount of methionine present is below that which has previously been suggested for adult dogs. There are indications that this reduction in methionine can lead to activation of favourable biochemical pathways, in particular, increased fatty acid oxidation and reduced de-novo lipogenesis, that may provide health advantages and/or promote longevity of the dog. In particular, the restricted methionine diet provided herein may alter flux through metabolic pathways and alter cellular processes and functions that subsequently support healthy ageing better than methionine rich diets. Measured levels of specific metabolites such as 5-methylthioadenosine, betaine, dimethylglycine, sphingosine-1-phosphate, taurocholate, taurochenodeoxycholate and 1-stearoyl-GPI as well as metabolic pathways (carnitine shuttle, pyrimidine metabolism, glycerophospholipid metabolism, glycine, serine and threonine metabolism nicotinate and nicotinamide metabolism and pyrimidine and purine metabolism) suggest that the present methionine restricted diets may have anti-aging effects and enhance longevity.

It has been generally recommended that a 15Kg dog requires a diet consisting of 1000kcal/day to maintain health. However, larger dogs need proportionally less calories per day/Kg bodyweight, due to a range of reasons including factors such as the difference in surface area:volume. A small dog will lose more heat proportionally, and therefore requires more energy to maintain their temperature. A coefficient of 0.75 is routinely used in the art to account for the weight/size differences across the species, and the Kg^{0.75} is defined as the "metabolic body weight".

However, irrespective of how many kcal/Kg metabolic bodyweight are used to formulate a diet, all diets are required to deliver all other nutrients in sufficient amounts. Thus, it is generally accepted that it may be necessary to provide nutrient supplements when feeding a low-calorie diet to a dog.

Applicant has found however, that the methionine levels may be reduced below those currently recommended. In accordance with the invention, these low levels are delivered to a dog, irrespective of the calorie intake, adjusted for the size of the dog, as the amounts are standardized to a 15 Kg dog. These figures may be translated directly to provide an amount of methionine to be provided in a diet aimed to provide a particular calorie level per kilogram metabolic body weight.

Applicant has now discovered that the biochemical pathways resulting in recycling of methionine are stimulated in adult dogs fed a low methionine diet. As a result, there may be no need to supply higher levels of cysteine to compensate for the sulphur reduction anticipated when feeding a low methionine diet.

In a particular embodiment, in order to avoid risks of a low protein or low sulphur amino acid diet, taurine is also included as an additional sulphur amino acid. The amount of taurine added is at least 0.125g/1000kcal, such as at least 0.25g/1000kcal, and suitably up to 0.5g/1000kcal, although higher amounts may be added if required. In particular, the composition comprises from 0.125-0.3g/1000kcal taurine. The addition of taurine allows the lowering of both methionine and cysteine in the diet without concerns regarding unacceptable reductions in the body taurine levels.

The weight percentage of methionine, cysteine and taurine required in order to provide the levels required by the diet composition of the invention will vary depending upon factors such as the nature of the composition and in particular the amount of water contained in it, as well as factors such as the calorific value of the other contents of the composition.

The diet composition of the invention will be suitable for dogs of all sizes and breeds.

For breeds and sizes of dogs that particularly prone to DCM, the diet suitably includes taurine as described above. Such breeds may include boxers, Dobermans, Great Danes, all of which are large dogs, having an average body weight in excess of 20Kg or 25Kg, or some spaniels such as cocker spaniels and English springer spaniels, which may be considered medium size dogs. The taurine containing embodiment of the composition of the invention may also be used with individuals of any breed that may be particularly prone to DCM. For example, some German Shepherds may be particularly prone to DCM.

Also described is a method of determining the size of the dogs by measuring their average body weight, and the diet composition of this invention is prescribed to those dogs having an average body weight in excess of 20kg or 25kg.

The diet composition of the invention will contain other nutrients in accordance with normal practice.

In a particular embodiment, the diet composition of the invention further comprises the nutrient choline. As described below, applicant has found that the low methionine diet can impact on blood choline levels and so it may be beneficial to add this as a dietary supplement. Where choline is added, it is suitably added in an amount of from 300 to 400mg/1000kcal, which will typically be in the range of from 1200 to 1600mg/Kg of the diet.

The diet composition may be in the form of an essentially dry food composition such as a kibble. The term "kibble" refers to a particulate pellet-like component of animal feeds, such as dog feeds. They may be hard or soft in texture, and will typically having a moisture, or water, content of less than 12% by weight. They are generally formed by an extrusion process. Once formed, the pellets may optionally be coated, for example with a substance that enhances the appeal of the kibble to the animal. Examples of such feed compositions are described for example in WO2011/091111.

Alternatively, the diet composition may comprise a wet food mix. Suitable wet food mixes may comprise more than 12% by weight of water. Wet food mixes may comprise semi-solid compositions wherein foodstuffs such as, or comprising, meat are suspended in gels or pastes. Wet food mixes are typically dispensed in tins.

Other wet food mixes may include mixtures of solids and liquids, for example a 'solids-in-gravy' type composition, as described for example in WO2017/009439. In such compositions, solid foodstuffs such as meat are suspended in a liquid gravy composition, and packaged. In this case, the composition may have a particularly high water content for example in excess of 85% by weight of water, in the overall composition.

Compositions of the invention will be prepared using conventional methods but with control of the levels of methionine, cysteine and, where present, taurine. For example, the components of the composition are selected to ensure that the required levels of methionine, cysteine and, if required taurine are present. However, this may require a complex selection of ingredients. Therefore, in a particular embodiment, compositions are prepared using ingredients which have a lower content of at least one of methionine, cysteine or taurine than that required by the invention, and the composition supplemented with methionine, cysteine and/or taurine during or after the preparation process so as to produce a diet composition of the invention. Suitably, the ingredients used will provide less than the required amount of at least two or of methionine, cysteine and where present taurine, and preferably less than the required amount of all of methionine, cysteine and taurine. In this way, suitable supplements of each amino acid may be added so that the final amount of these amino acids in the composition is closely controlled.

Use of vegetable proteins which are naturally low in sulphur-containing amino acids is a particularly convenient way of achieving this.

Thus a diet is devised to fulfill the requirements of the present invention by assimilating ingredients, including proteins, in particular where at least some of the protein is vegetable proteins, determining the total content of methionine, cysteine and taurine present in the ingredients, and adding sufficient methionine, cysteine or a cysteine providing derivative thereof, and/or taurine to form a diet composition in accordance with the invention.

Methionine and cysteine and where present also taurine are suitably supplied as free amino acids, or in the case of cysteine, a cysteine-providing derivative thereof, as described above. However, they may be supplied in the form of other natural ingredients, including in particular proteins, to ensure that a composition of the invention is formed.

Diets will contain additional ingredients such as oils, fats, fiber, and fatty acids (such as omega-3 and omega-6), as would be understood by one of ordinary skill in the art. With the exception of providing less than the recommended amount of methionine, and in some embodiments, added taurine, the diet compositions provided herein may be considered complete and balanced diets, suitable to feeding a dog as their main source of caloric intake. The present diet compositions are not formulated as supplements.

Minerals such as calcium, phosphorus, iron, iodine, copper, manganese and zinc, some of which may be supplied in the form of crude ash, may also be included.

In addition, antioxidants including vitamins such as vitamin E, vitamin A and vitamin D may be provided.

In a particular embodiment, wherein the other ingredients are derived largely from vegetable proteins as described above, the diet composition may be further supplemented with vitamin D, calcium, and L-carnitine.

Diets of the invention contain sufficient methionine to support metabolism and may provide health benefits. In particular, the diets may impact on blood lipid levels and lower cholesterol, changes which are associated with longevity. Thus, the diets may prolong lifespan whilst minimizing the risk of taurine deficiency.

Applicant has discovered a dietary range of methionine that is lower than current (recommended) dietary inclusion levels, that meets the requirements for the dog, is safe to consume, and that produced biological responses believed to lead to improved health and/or increased lifespan. These include specific metabolites such as 5-methylthioadenosine, betaine, dimethylglycine, sphingosine-1-phosphate, taurocholate, taurochenodeoxycholate and 1-stearoyl-GPI as well as metabolic pathways (carnitine shuttle, pyrimidine metabolism, glycerophospholipid metabolism, glycine, serine and threonine metabolism nicotinate and nicotinamide metabolism and pyrimidine and purine metabolism).

Thus, also described is a method for promoting longevity in a dog, said method comprising feeding the dog a diet as described above. Methods of treating dilated cardiomyopathy, or preventing dilated cardiomyopathy in dogs prone to development of the same, are also provided.

Large and giant breed dogs are particularly susceptible to development of DCM and thus, may find particular benefit from the method. Such breeds may include boxers, Dobermans, Great Danes, all of which are large dogs, having an average body weight in excess of 20Kg or 25Kg, or some spaniels such as cocker spaniels and English springer spaniels, which may be considered medium size dogs. The taurine containing embodiment of the composition of the invention may also be used with individuals of any breed that may be particularly prone to DCM. For example, some German Shepherds may be particularly prone to DCM.

Also described is is a method of determining the size of the dogs by measuring their average body weight, and the diet composition of this invention is prescribed to those dogs having an average body weight in excess of 20kg or 25kg.

### Example 1

### Determination of the Effect of Methionine Restriction in Dog Diets

### Animals

Adult Labrador retrievers (n= 54) were used in this study. Dogs were housed and exercised with other dogs in their dietary group throughout the whole trial.

### Diets

A pelleted semi-purified diet (Ssniff Speziäldiaten Gmbh) was utilized to enable manipulation of methionine and cysteine content whilst keeping other nutrients constant. All diets were nutritionally complete, iso-caloric and iso-nitrogenous (through altering alanine content) and were fed to maintain an ideal body condition score.

### Study design

Dogs were placed in to three dietary groups A, B or C balanced for age and gender. All dogs were fed an appropriate diet, with methionine at 1.37g/1000kcal and cysteine at 1.31 g/1000kcal cysteine, for 4 weeks prior to the first set of samples to provide a baseline before changing to their allocated diet.

The diets that were then fed were similar in all respects other than the methionine/cysteine content which was as set out in Table 1.

**Table 1**

| **Diet** | **Methionine (g /1000kcal)** | **Cyst(e)ine (g/ 1000kcal)** |
|---|---|---|
| **A** | 1.37 | 1.31 |
| **B** | 0.55 | 2.13 |
| **C** | 0.71 | 1.97 |

In diets B and C, methionine level was reduced but the level of cysteine was increased to ensure that the recommended total level of sulphur amino acids was maintained.

Dogs in each group were fed the respective diet for a total of 32 weeks.

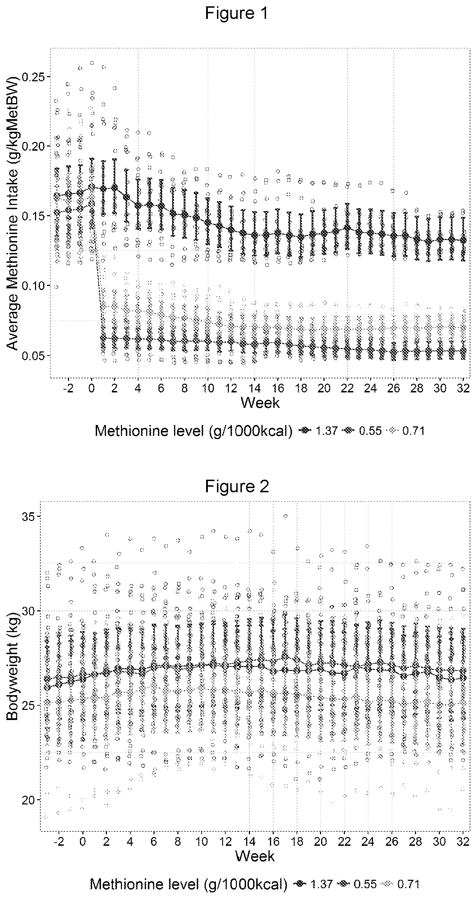

The dogs were initially fed a sufficient amount of each diet to provide for their individual needs and to maintain body condition score (BCS) based on calorie intake. Dogs that started to gain weight (Figure 2) had their daily intake adjusted accordingly to maintain BCS. As a result, average daily intake (kcal/KgMetBW) significantly reduced over the study period for all dietary groups (p<0.001) irrespective of methionine level. This may be due to the diet being very digestible and having more accessible calories.

Analysis of average daily methionine intake levels (Figure 1) found no significant alteration in methionine intake levels in the first 4 weeks of the study (weeks -3 to 0) as expected by the study design. In week 4 dogs transitioned to diets containing differing methionine contents. Analysis of individual contrasts from weeks 1 to 32 revealed a significant difference in methionine intake between all three dietary groups (p<0.001). Analysis within the dietary groups revealed that methionine intake significantly reduced from week 7 to 32 for the 1.37g/1000kcal methionine group when compared to baseline.

Dogs were sampled every 8 weeks (week 8, 16, 24 & 32) to monitor any changes in taurine levels (primary outcome) throughout the study. Fasted blood samples (8.7ml) were aliquoted into 5 different tubes (either Lithium Heparin or EDTA).

### Whole blood taurine analysis

Whole blood was aliquoted and frozen at -20°C. Freeze-thaw cycling was used to lyse the red blood cells. Once thawed, sterile water was added (1:4). Sample processing buffer (5% SSA, 500µMol/L Norleucine and D-Glucosaminic acid lithium loading buffer Biochrom) was added at a 1:2 dilution in order for analysis to be normalized, positively charge the amino acids and also to remove protein from the sample. The sample was mixed and incubated at room temperature for 20 minutes, before being centrifuged (5 minutes at 4°C at 10,000rpm). The supernatant was transferred to a Whatman Mini-Uniprep Syringeless 0.2µm filter vial to filter the sample. The final sample was diluted 1:8, and injected into the instrument in duplicate. The analysis was carried out using a Biochrom 30+ Analyzer.

### Plasma Biochemistry

Whole blood samples were treated to provide plasma for routine analysis on an AU480 Chemistry Analyzer (Beckman Coulter).

### Statistics

The primary response variable was the whole blood taurine and the secondary supportive response variables were plasma taurine, urinary taurine, biochemistry and haematology measures and energy/caloric intake. For each response measured, linear mixed model analysis (REML) was used to allow for repeated measures on each dog over the course of the study.

### Results

### Whole Blood Taurine

The whole blood taurine levels significantly decreased (p<0.001) overtime in all dietary groups. However, no difference between either methionine restricted groups (0.55 or 0.71g/1000kcal methionine) compared to the control group (1.37g/1000kcal methionine) was noted.

### Bodyweight

Bodyweight (Kg) was not affected by the methionine content of the diet (Figure 2), as no significant differences were noted between dietary groups at any time point. A significant increase in bodyweight over the duration of the study (p<0.001) was noted within dietary groups when compared to baseline bodyweight, with Diet A (1.37 g/1000kcal methionine) body weight significantly increased at week 1, weeks 3-15 and week 23 compared to baseline bodyweights. In addition, body weight within the dietary group fed Diet B (0.55 g/1000kcal methionine) was significantly increased at weeks 14-15 and weeks 17-18 while body weight within the dietary group fed Diet C (0.71 g/1000kcal methionine) significantly increased at week 6 when compared to baseline bodyweights.

### Cholesterol

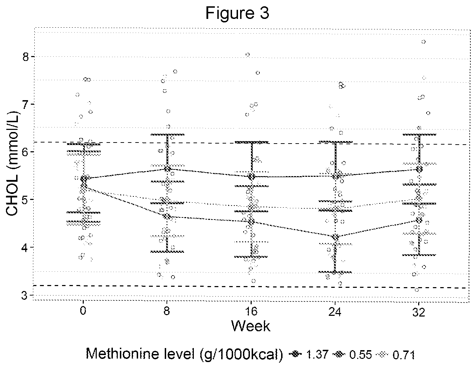

Cholesterol levels were significantly reduced (p<0.001) in the Diet B group (0.55g/1000kcal methionine) compared to the Diet A control group (1.37g/1000kcal methionine) at weeks 24 and 32 (Figure 3). Analysis within dietary groups revealed that for the Diet B group (0.55g/1000kcal methionine) cholesterol levels were significantly reduced (p<0.001) compared to baseline at 8, 16, 24 and 32 weeks. This suggests that Diet B had an impact on lipid metabolism.

### Conclusion

Thus, though restricted methionine Diets B and C (containing 0.55 and 0.71g/1000kcal methionine, respectively) represent a methionine restriction of 52% and 38% respectively from current recommended levels, the feeding of Diets B and C had no significant effect upon free circulating taurine (whole blood taurine) when compared to control Diet A (1.37g/1000kcal).

Although no significant difference in taurine levels was noted between dietary groups throughout this study, a significant effect of time was evident, with circulating taurine decreasing over the study period when compared to baseline levels in all dietary groups. It is likely that the decreases in taurine levels associated with all dietary groups are likely a consequence of the reduced total sulphur amino acid intake associated with all diets over the study period in order to maintain ideal body condition.

No adverse effect upon taurine synthesis was noted when reducing the methionine level from 1.37 to 0.71 (Diet C) or 0.55g (Diet B) /1000kcal for a seven-month period, which suggests that diets containing these reduced methionine levels are safe to feed to dogs. Although no adverse effects upon blood biochemistry and hematology were noted during the 32 week duration of this study, perturbations to lipid profiles (see, example 2) suggest alterations to fatty acid oxidation and de-novo lipogenesis.

The restriction of methionine within this study significantly reduced both cholesterol (24 & 32 weeks) and triglyceride (24 weeks) levels in the 0.55g (Diet B)/1000kcal group compared to controls. The reduced triglyceride and cholesterol levels associated with methionine reduction in this study are thought to be a consequence of increased fatty acid oxidation and reduced de-novo lipogenesis.

### Example 2

### Metabolomic Analysis of the Samples obtained in Example 1

A full metabolomic analysis of plasma samples was undertaken with respect to the samples obtained from dogs fed on diets A and B in table 1, which are hereinafter referred to as 'control' and 'test' samples respectively. Plasma samples were prepared (within 1 hour of sample) and stored at -80°C until sent on dry ice to Metabolon Inc. for analysis, using Ultra High Performance Liquid chromatography with tandem Mass Spectrometry (UHPLC/MS/MS) and Gas chromatography/ Mass Spectrometry (GC/MS).

For each metabolite the mean of all samples was assigned a value of 1, with each sample being assessed as a fold-change related to this so a value of 0.75 represents a 25% reduction, whilst a value of 1.5 is a 50% increase.

Individual metabolites significantly different between baseline and 32 weeks in the Test group but not the control group were noted (Figure 4). In general, most of the non-lipids in this figure tended to increase over the study whilst the lipid metabolites tended to decline. Exceptions to this simplified observation of decreasing lipid pools included 1-stearoyl-GPI, docosatrienoate (22:3n6, sphingosine-1-phosphate, taurocholate and taurochenodeoxy cholate. It is believed that these decreases indicate that the diet composition is capable of providing an anti-aging effect.

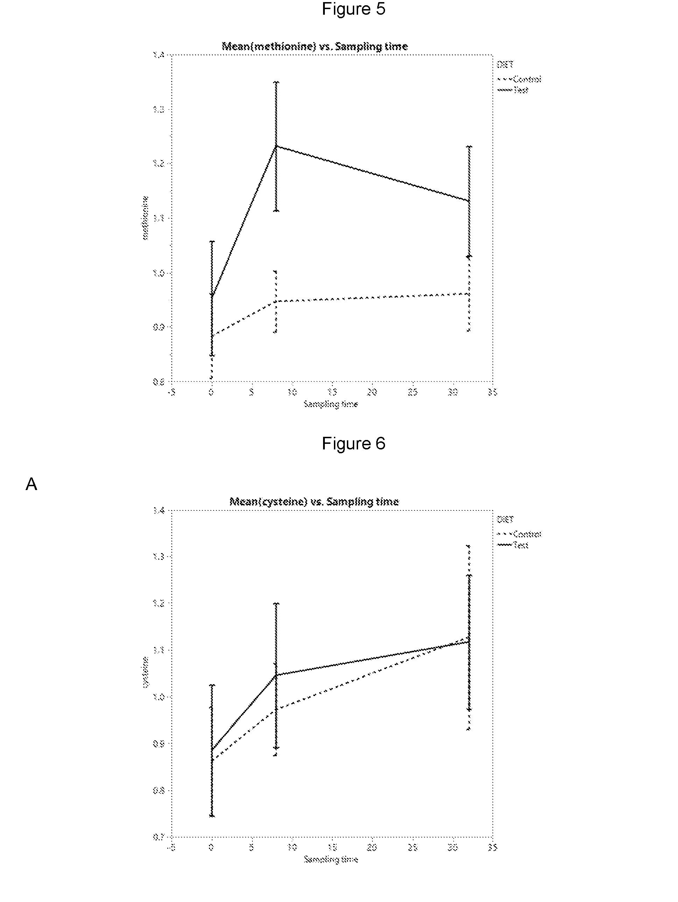

Despite reduced dietary methionine intake, the fasted plasma methionine pool increased 1.3-fold at 8 weeks and remained higher than at baseline and control diet at 32 weeks (Figure 5).

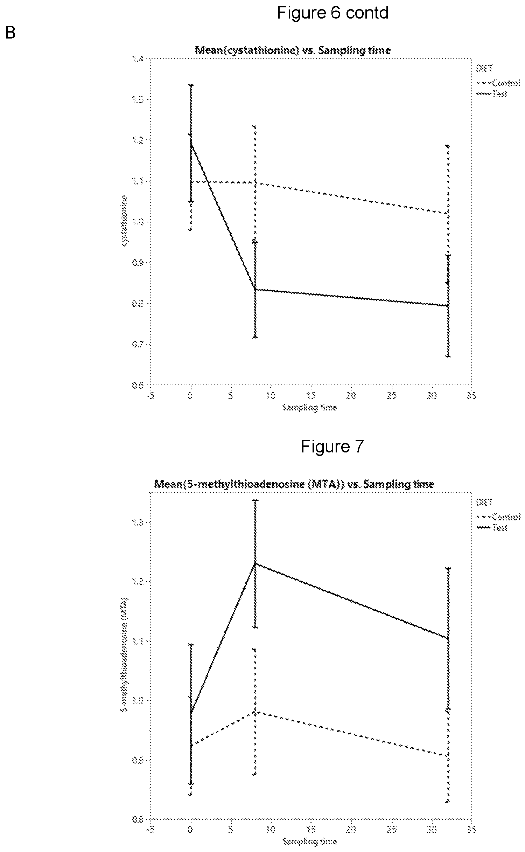

Whilst dietary cysteine (as cystine) intake increased in the test group, fasted plasma cysteine increased significantly in both groups and so remained non-significantly different between groups. In fasted plasma, there was no significant effect of the increase in dietary cystine intake on the precursor, cystathionine at any time point (Figure 6A-6B). This reduction may simply reflect a lower demand for cysteine synthesis on the supplemented diet.

The results suggest that the supplementary cysteine used in the experiment is not an essential dietary requirement. As a result, a suitable diet may comprise less cysteine and in particular, approximately equivalent amounts of cysteine and methionine.

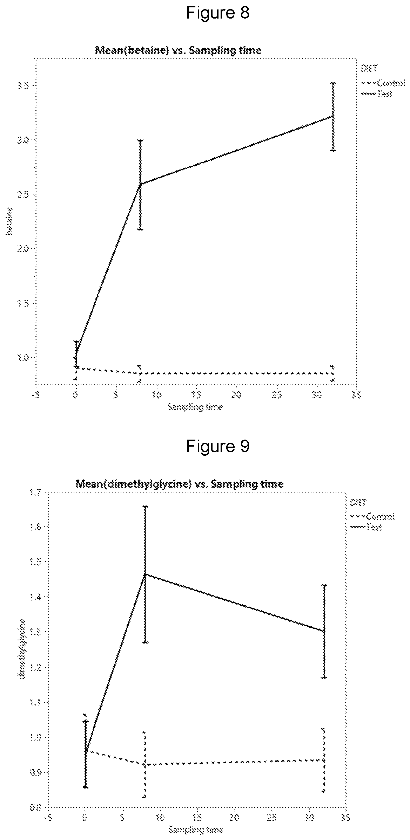

Betaine synthesis predominantly takes place in the liver, within mitochondria. The plasma pool betaine levels showed an increase of 3-fold and 3.8 fold at 8 and 32 weeks respectively (Figure 8). These data are consistent with the lower dietary methionine requiring gradually more choline to be diverted to betaine to support methionine cycling.

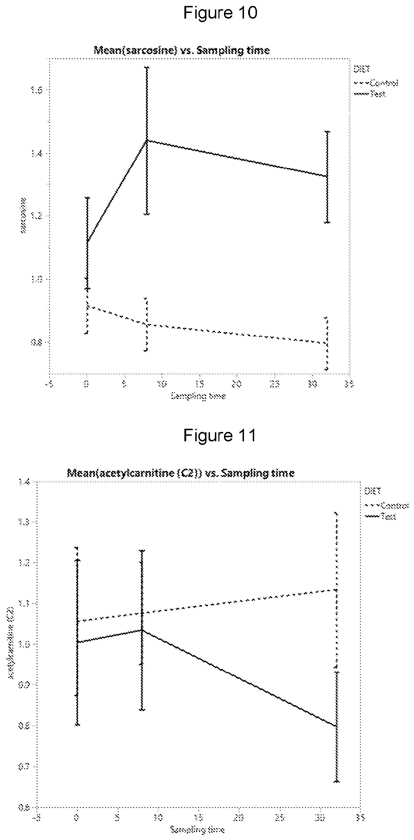

It is of note that both dimethylglycine and sarcosine increased (up to 1.6 and 1.7-fold respectively; Figures 9 and 10) are consistent with increased choline oxidation and betaine-homocysteine methyltransferase (BHMT) activity to support methionine recycling increasing their plasma pool at higher rates of production.

The observation that choline oxidation is required to support the methionine plasma pool indicates that the dietary cystine, added to maintain the total sulphur amino acid content in the diet, was, as expected, not functionally equivalent to support the plasma methionine pool. As such, this indicates that the total sulphur amino acid value may be reduced, as long as the methionine requirement is achieved.

No difference between diet groups was observed for presumed indicator metabolites of methionine availability. Metabolites considered most likely to be affected if methionine availability was negatively affected include taurine (the primary output of the study is the measurement of whole blood taurine) and the dominant sinks for SAM-derived methyl groups, creatine and phosphatidylcholine (via Phosphatidylethanolamine N-methyltransferase (PEMT)-activity).

Plasma taurine was not significantly different between diet groups at any time point. Creatine levels were not affected during this study, indicating that methionine cycle was sufficient to support the plasma pools of this output from SAM synthesis.

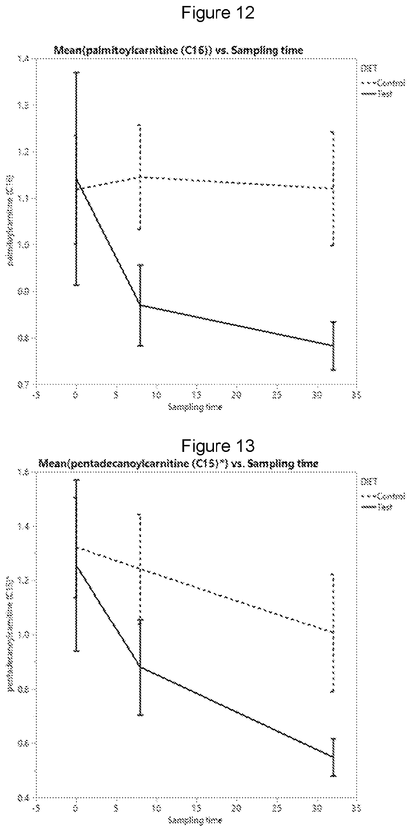

Pentadecanoylcarnitine, an odd-chained saturated fatty acid (C15:0), increased in this study (Figure 13), which is indicative of α-oxidation and previously inversely associated with various diseases. Furthermore, acetylcarnitine and palmitoylcarnitine, two of the top 10 metabolites that have been found to be increased in the liver metabolome, decreased in this study (Figures 11 and 12).

In addition, significant results were noted with respect to polyamine synthesis. In particular, 5-methylthioadenosine (5-MTA) levels varied as shown in Figure 7.

Metabolite Set Enrichment Analysis (MSEA) of these data identified pathways differing significantly between diet groups at 32 weeks and also pathways that differed in rate of change between diet groups between base and 32 weeks. MSEA determines an enrichment score based on the number of metabolites within a pathway that are detected (m) and how many of these are significant (k) compared with the likelihood based on the total number of significant metabolites proportional to the total number of metabolites detected. An enrichment value higher than 1 would indicate higher than expected by change. All pathways with an enrichment score > 1.5 and with > 1 member detected are listed in Table 2 hereinafter.

These results suggested that despite lower dietary methionine, increased methionine may indicate activities to increase flux through the methionine cycle. In particular, changes in lipid metabolism may be the results of diversion of choline from lipid metabolism, including reducing availability of S-adenyosyl-methionine for PEMT activity. Changes in nucleotide, cofactor and histidine metabolism may support methionine re-synthesis. As plasma methionine was increased, lower dietary methionine may provide some longevity benefits indirectly though remodelling lipid metabolism and diverting 1C from other pathways.

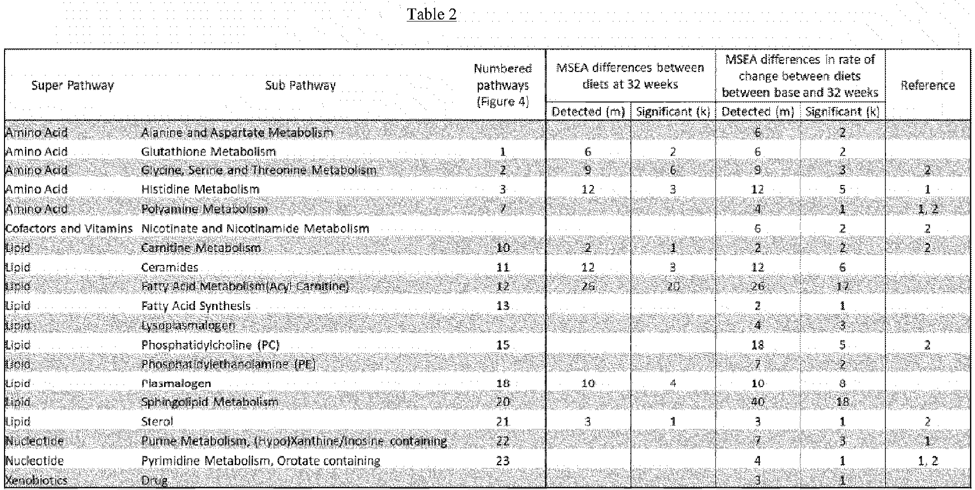

Reported in longevity-related studies:¹ Ghosh et al., (2017), PLOS ONE 12(5) e0177513; ² Green et al., (2017), Aging Cell; Volume 16, Issue 3 p529-540.

## Claims

1. A diet composition for an adult dog, said composition comprising a combination of methionine and cysteine, and wherein:
a. the amount of methionine present is 0.55 g/1000kcal and wherein the amount of cysteine present is 2.13 g/1000kcal; or
b. the amount of methionine present is 0.71 g/1000kcal and wherein the amount of cysteine present is 1.97 g/1000kcal.

2. A diet composition according to claim 1a, wherein the diet composition is formulated to be fed at 95kcal/Kg body weight.

3. A diet composition according to any one of the preceding claims which is in the form of a pelleted composition.

4. A diet composition according to any one of claims 1 to 3 for use in a method for treating a dog that is prone to dilated cardiomyopathy, said method comprising feeding the dog said diet composition.

5. The diet composition of claims 1 to 3 for use in the treatment of a dog that is prone to dilated cardiomyopathy.

## Patentansprüche

1. Diätzusammensetzung für einen erwachsenen Hund, wobei die Zusammensetzung eine Kombination aus Methionin und Cystein umfasst und wobei:
a. die Menge an Methionin, die vorhanden ist, 0,55 g/1000 kcal ist und wobei die Menge an Cystein, die vorhanden ist, 2,13 g/1000 kcal ist; oder
b. die Menge an Methionin, die vorhanden ist, 0,71 g/1000 kcal ist und wobei die Menge an Cystein, die vorhanden ist, 1,97 g/1000 kcal ist.

2. Diätzusammensetzung gemäß Anspruch 1a, wobei die Diätzusammensetzung formuliert ist, um mit 95 kcal/kg Körpergewicht gefüttert zu werden.

3. Diätzusammensetzung gemäß einem der vorhergehenden Ansprüche, die in der Form einer pelletierten Zusammensetzung ist.

4. Diätzusammensetzung gemäß einem der Ansprüche 1 bis 3 zur Verwendung in einem Verfahren zum Behandeln eines Hundes, der anfällig für dilatative Kardiomyopathie ist, wobei das Verfahren Füttern des Hundes mit der Diätzusammensetzung umfasst.

5. Diätzusammensetzung nach Anspruch 1 bis 3 zur Verwendung bei der Behandlung eines Hundes, der anfällig für dilatative Kardiomyopathie ist.

## Revendications

1. Composition de régime alimentaire pour chien adulte, ladite composition comprenant une combinaison de méthionine et de cystéine, et dans laquelle :
a. la quantité de méthionine présente est de 0,55 g/1 000 kcal et dans laquelle la quantité de cystéine présente est de 2,13 g/1 000 kcal ; ou
b. la quantité de méthionine présente est de 0,71 g/1 000 kcal et dans laquelle la quantité de cystéine présente est de 1,97 g/1 000 kcal.

2. Composition de régime alimentaire selon la revendication 1a, dans laquelle la composition de régime alimentaire est formulée pour être donnée en alimentation à 95 kcal/Kg de poids corporel.

3. Composition de régime alimentaire selon l'une quelconque des revendications précédentes qui se présente sous la forme d'une composition granulée.

4. Composition de régime alimentaire selon l'une quelconque des revendications 1 à 3, destinée à être utilisée dans un procédé de traitement d'un chien sujet à une cardiomyopathie dilatée, ledit procédé comprenant l'alimentation du chien avec ladite composition de régime alimentaire.

5. Composition de régime alimentaire selon les revendications 1 à 3 destinée à être utilisée dans le traitement d'un chien sujet à une cardiomyopathie dilatée.
